Europäisches Patentamt

European Patent Office

Office européen des brevets

(19)

(11) **EP 0 964 669 B1**

(12) **FASCICULE DE BREVET EUROPEEN**

(45) Date de publication et mention
de la délivrance du brevet:
**13.11.2002   Bulletin 2002/46**

(51) Int Cl.⁷: **A61K 7/16**

(21) Numéro de dépôt: **97934601.2**

(86) Numéro de dépôt international:
**PCT/FR97/01349**

(22) Date de dépôt: **21.07.1997**

(87) Numéro de publication internationale:
**WO 98/003153 (29.01.1998 Gazette 1998/04)**

(54) **SILICE COMPATIBLE AVEC LES AROMES, SON PROCEDE DE PREPARATION ET COMPOSITIONS DENTIFRICES LA CONTENANT**

GESCHMACKSVERTRAEGLICHES KIESELGEL, VERFAHREN ZU SEINER HERSTELLUNG UND DIESE ENTHALTENDE ZAHNPASTEZUSAMMENSTELLUNG

FLAVOUR-COMPATIBLE SILICA, METHOD FOR PREPARING SAME AND TOOTHPASTE COMPOSITIONS CONTAINING SAID SILICA

(84) Etats contractants désignés:
**BE DE ES FI FR GB NL**
Etats d'extension désignés:
**RO SI**

(30) Priorité:  **23.07.1996  FR 9609202**

(43) Date de publication de la demande:
**22.12.1999   Bulletin 1999/51**

(73) Titulaire: **RHODIA CHIMIE**
**92408 Courbevoie Cédex (FR)**

(72) Inventeurs:
 • **DROMARD, Adrien**
  **F-69006 Lyon (FR)**

 • **VIOT, Jean-François**
  **F-69540 Irigny (FR)**

(74) Mandataire: **Fabre, Madeleine-France**
 **Rhodia Services,**
 **Direction de la Propriété Industrielle,**
 **40, rue de la Haie-Coq**
 **93306 Aubervilliers Cedex (FR)**

(56) Documents cités:
 **EP-A- 0 275 706          WO-A-93/23007**
 **WO-A-94/06868          FR-A- 2 271 840**
 **US-A- 4 340 583**

**Description**

La présente invention a pour objet un procédé de préparation d'une silice de compatibilité améliorée avec les arômes, utilisable notamment comme agent abrasif et/ou épaississant dans les pâtes et gels dentifrices, ainsi que l'application de cette silice comme agent abrasif et/ou épaississant dans les pâtes et gels dentifrices ou autres formulations pour hygiène dentaire ou buccale.

Les silices précipitées et les gels de silice sont largement utilisés comme abrasifs dans les pâtes dentifrices. Comparativement aux autres abrasifs traditionnellement utilisés, tels que les phosphates de calcium ou les carbonates de calcium précipités, les silices précipitées et les gels de silice possèdent les propriétés et performances spécifiques suivantes :

- une grande compatibilité avec les agents thérapeutiques, le fluor en particulier, mais aussi les agents anti-tartre
- un indice de réfraction finement réglable, pouvant aller de 1,44 à 1,46 selon qu'il s'agit de silice précipitée ou de gel de silice, autorisant leur application dans les formulations de gels translucides.

Par ailleurs, les silices précipitées présentant les volumes poreux les plus élevés et les gels de silice sont utilisés en tant qu'agents épaississants des pâtes et gels dentifrices. Ils apportent plus particulièrement un seuil d'écoulement élevé et diminuent considérablement le filant des produits dentifrices.

Les silices précipitées abrasives ou épaississantes et les gels de silice sont tout à fait compatibles avec les agents thérapeutiques et permettent aussi la fabrication de gels translucides.

Par contre, il est reconnu que la présence de silice précipitée ou de gel de silice dans les formulations dentifrices modifie la restitution des arômes utilisés. Il est également connu que l'origine de cette modification de la restitution des arômes réside dans l'adsorption des molécules d'arômes sur la surface de la silice. Cette adsorption est vérifiée avec la plupart des arômes utilisés dans les dentifrices.

Il a été proposé de minimiser l'interaction des silices avec les arômes par traitement des silices à l'état de produits finis par un matériau minéral alcalin (hydroxyde sodium par exemple). Une réduction de 2% de l'aire du pic "headspace" (espace de tête) de l'arôme mesurée par chromatographie gazeuse, contre une réduction de 20% dans le cas d'une silice non traitée est montrée (WO 94/06868).

Il est aussi connu de revêtir la surface des silices abrasives par un polymère cationique, afin de rendre ces dernières moins adsorbantes vis-à-vis des agents cationiques thérapeutiques (US-A-4 157 387).

Un premier objet de l'invention consiste en un procédé de préparation de la silice de compatibilité améliorée avec les arômes présentant pour une abrasivité RP/DA ("RHONE-POULENC Dentine Abrasion") inférieure ou égale à 10, une compatibilité avec les arômes supérieure à 10%, de préférence supérieure à 15%, et pour une abrasivité RP/DA ("RHONE-POULENC Dentine Abrasion") supérieure à 10, une compatibilité avec les arômes d'au moins 30%, de préférence supérieure à 50%, par traitement, à l'aide d'un composé organique, de la surface d'une silice issue de la réaction d'un silicate de métal alcalin avec un agent acide minéral ou organique, ledit procédé étant caractérisé en ce que ledit composé organique est non-ionique et en ce que ledit traitement est réalisé par développement de liaisons hydrogène entre les groupements silanols SiOH de la surface de la silice à traiter et ledit composé organique non-ionique, ladite silice traitée étant ensuite si nécessaire séparée et/ou lavée et/ou séchée.

La compatibilité de la silice vis-à-vis des arômes est mesurée par chromatographie "espace de tête" (headspace) à l'aide d'un chromatographe en phase gazeuse VARIAN 3400, de la manière suivante :

L'arôme choisi est la L-carvone, qui constitue une part importante des arômes élémentaires traditionnels ; celui-ci est mis directement en contact avec la silice à tester, dans un flacon "espace de tête". Après fermeture du flacon et mise à l'équilibre thermodynamique entre la phase solide et la phase vapeur environnante, à l'aide d'un bain thermostaté à 50°C, on prélève 1ml de phase vapeur, que l'on injecte dans la colonne du chromatographe.

Le chromatogramme obtenu représente la composition de la phase vapeur ; on mesure l'aire du pic chromatographique principal de la L-carvone.

Une mesure de référence est réalisée dans les mêmes conditions, en remplaçant la silice par du phosphate dicalcique (VICTOR DF de RHONE-POULENC), connu pour sa bonne compatibilité avec les arômes.

La compatibilité de la silice pour les arômes, CA, est donnée par l'équation suivante :

$$CA = \frac{\text{aire du pic correspondant à la L-carvone en présence de silice}}{\text{aire du pic correspondant à la L-carvone en présence de phosphate dicalcique}} \times 100$$

L'évaluation de l'abrasivité RP/DA est effectuée selon un test permettant de quantifier l'abrasivité relative d'un abrasif ou d'une pâte dentaire à l'issue d'un cycle de brossage sur de la dentine humaine préalablement polie, par rapport à une poudre de silice de référence de grade dentaire ; l'abrasivité absolue de la poudre ou de la pâte testée est déterminée à l'aide d'un profilomètre TALYSURF 10 de RANK TAYLOR HOBSON, qui mesure les profondeurs moyennes d'usure de la dentine après brossage. Une série de dents humaines est enrobée de résine de polyméthacrylate de méthyle puis polie à l'aide d'une polisseuse jusqu'à apparition de la dentine.

On isole sur les éprouvettes deux zones latérales par collage d'adhésif transparent ; les parties de la dentine protégées constitueront lors de la mesure profilométrique, les surfaces de référence à partir desquelles est mesurée la profondeur moyenne de la trace d'usure.

Le brossage des surfaces est effectué à l'aide d'un abrasimètre équipé de cuves supports et muni de brosses à dents ; les conditions de brossage sont de 1500 aller-retours ; chaque produit testé est précédé et suivi d'un cycle de brossage et de mesures avec l'abrasif de référence.

Les dispersions de poudre ou de pâte à tester sont préparées selon la procédure recommandée par l'American Dental Association et décrite par John J. HEFFEREN dans J. Dental Research, Vol 55 No.4, 563-573, 1976.

En ce qui concerne les poudres (celles à tester, de même que celle de référence), 80g de poudre sont dispersées, à l'aide d'un agitateur RAYNERI, dans un milieu constitué de glycérol (10%), carboxyméthylcellulose (0,5%) et d'eau distillée 89,5%.

En ce qui concerne les pâtes, 200g de pâte sont dispersés, à l'aide d'un agitateur RAYNERI, dans 320ml d'eau distillée.

On réalise par dentine 4 mesures profilométriques dans une zone située vers un bord défini de l'échantillon ; le test d'un même produit sur 4 dentines fournit au total 16 valeurs pour le calcul de la profondeur moyenne d'usure $\underline{Hm}$.

L'abrasivité relative RP/DA (%) du produit testé est donné par le rapport suivant

$$Rp/DA \ (\%) = \frac{Hm \text{ obtenue avec le produit testé}}{Hm \text{ obtenue avec le produit de référence}} \ x118$$

la valeur 118 correspondant à l'abrasivité RDA de la silice de référence, l'abrasivité RDA ("Radioactive Dentine Abrasion") étant mesurée selon la méthode décrite par J.J. HEFFERREN dans "Journal of Dental Research", vol. 55(4), page 563, 1976.

La silice de l'invention peut aussi bien être une silice de précipitation qu'un gel de silice.

L'obtention de silice de précipitation ou de gel de silice par réaction d'un silicate de métal alcalin avec un agent acide minéral ou organique est une opération bien connue en soi.

Selon le schéma classique, ledit silicate est de préférence mis en oeuvre sous forme d'une solution aqueuse de silicate de métal alcalin M (le métal M représentant notamment le sodium ou le potassium) de rapport $SiO_2$ / $M_2O$ de l'ordre de 2 à 4 ; cette solution présente généralement une concentration en $SiO_2$ de l'ordre de 30 à 350g/l. L'agent acide peut être tout acide minéral ou organique fort tel que l'acide sulfurique, l'acide nitrique, l'acide chlorhydrique, l'acide acétique, l'acide formique, l'acide carbonique ... ; généralement celui-ci est également mis en oeuvre en solution aqueuse à une concentration de l'ordre de 60 à 400g/l dans le cas de l'acide sulfurique.

Le plus fréquemment cette opération est réalisée à une température de l'ordre de 80 à 100°C, en mettant en oeuvre comme silicate, du silicate de sodium de rapport $SiO_2$ / $Na_2O$ de l'ordre de 2 à 4, et comme agent acide de l'acide sulfurique.

Les quantités respectives de silicate de sodium et d'acide sulfurique sont ajustées de façon à maintenir le pH du milieu réactionnel de formation de silice à une valeur sensiblement constante de l'ordre de 2 à 10, plus précisément de l'ordre de 2 à 5 pour obtenir un gel de silice et de 5 à 10 pour obtenir une silice précipitée.

La bouillie réactionnelle obtenue est ensuite filtrée et lavée généralement à l'eau, puis le gâteau de filtration récupéré est séché et éventuellement broyé.

Un avantage de l'invention consiste en ce que, si le traitement est réalisé sur une suspension de silice, celui-ci peut avoir lieu au cours du procédé d'obtention même de la silice.

Selon un premier mode de réalisation de l'invention, la silice à traiter se présente au sein de la suspension diluée constituée par la bouillie issue directement de la réaction du silicate de métal alcalin avec l'agent acide minéral ou organique.

Cette bouillie réactionnelle est constituée d'une suspension de silice dans une solution de sel (le plus généralement une solution de sulfate de sodium).

La concentration de la silice, exprimée en sec, au sein de ladite suspension peut aller de 10 à 150 g, généralement de 40 à 80g de $SiO_2$ par litre de solution de sel.

Selon ce premier mode de réalisation de l'invention, le traitement par le composé organique peut être réalisé par mise en contact, sous agitation mécanique, de la bouillie réactionnelle et du composé organique de traitement, à une température de l'ordre de 20°C à 100°C, à un pH de l'ordre de 1 à 10, de préférence de l'ordre de 3 à 6.

La bouillie réactionnelle, ainsi traitée, est ensuite séparée par filtration, par tout moyen connu, puis lavée à l'eau ; le gâteau de silice obtenu peut, éventuellement après délitage, être ensuite séché afin de présenter un taux d'humidité (mesuré à 105°C selon la Norme ISO 787/2) inférieur à 50%, de préférence inférieur à 10%, tout particulièrement de l'ordre de 4 à 8%, puis broyé si nécessaire par tout moyen jusqu'à l'obtention du produit fini de granulométrie désirée.

Selon un deuxième mode de réalisation de l'invention, la silice à traiter se présente au sein de la suspension concentrée constituée par le gâteau de silice issu de la filtration, avec éventuellement lavage à l'eau, de la bouillie réactionnelle formée par réaction du silicate de métal alcalin avec l'agent acide minéral ou organique.

L'étape de filtration de la bouillie réactionnelle peut être réalisée par tout moyen connu, par exemple à l'aide d'un filtre presse, d'un filtre sous vide ...

Le gâteau est éventuellement lavé, généralement à l'eau.

Ledit gâteau est constitué d'une suspension de silice dans de l'eau pouvant contenir une faible quantité de sel résiduel. La concentration de la silice, exprimée en sec, dans ledit gâteau est généralement telle que ledit gâteau présente un extrait sec (mesure de la perte au feu à 1000°C, selon la Norme ISO 3262/11) de l'ordre de 10 à 45%.

Le traitement, par le composé organique, de la silice présente au sein du gâteau de filtration peut être réalisé par mise en contact, sous agitation mécanique, du gâteau de filtration et du composé organique de traitement, à une température de l'ordre de 10°C à 80°C, de préférence de l'ordre de 15°C à 40°C, à un pH de l'ordre de 3 à 10, de préférence de l'ordre de 5 à 8.

Le gâteau de silice traité obtenu peut, éventuellement après délitage, être ensuite séché, afin de présenter un taux d'humidité inférieur à 50%, de préférence inférieur à 10%, de préférence de l'ordre de 4 à 8%, puis broyé si nécessaire par tout moyen jusqu'à l'obtention du produit fini de granulométrie désirée.

Selon un troisième mode de réalisation de l'invention, la silice à traiter par le composé organique est une silice sous forme sèche, pouvant être une silice précipitée ou un gel de silice, présentant une humidité inférieure à 50%, de préférence inférieure à 10%, de préférence de l'ordre de 4 à 8%, obtenue par séchage, éventuellement après délitage, du gâteau de silice issu de la filtration avec lavage de la bouillie réactionnelle formée par réaction du silicate de métal alcalin avec l'agent acide minéral ou organique. Le traitement par le composé organique peut être effectué par pulvérisation du composé organique de traitement sur la surface de la silice sèche, cette opération de pulvérisation étant de préférence réalisée soit sur ladite silice prébroyée soit au cours d'une opération de broyage de ladite silice. Ledit composé organique à pulvériser peut se présenter sous forme d'une solution aqueuse contenant de l'ordre de 0,5 à 10% de matière active, la quantité de solution absorbée pouvant représenter de 0,1 à 4 fois le poids de silice concerné ; la silice traitée est ensuite éventuellement séchée pour obtenir la siccité voulue ; cette opération de séchage peut être réalisée en lit fluidisé.

Il est tout particulièrement intéressant de mettre en oeuvre ce troisième mode de réalisation en préliminaire de la préparation de la formulation dentifrice. La silice traitée peut alors ne pas être séchée.

Le composé à pulvériser peut également être pulvérisé sous forme fondue s'il n'est pas naturellement liquide à la température de l'opération.

Selon un quatrième mode de réalisation de l'invention, la silice à traiter se présente au sein d'une suspension concentrée obtenue par redispersion dans l'eau d'une silice sèche préalablement préparée, silice sèche pouvant être une silice précipitée ou un gel de silice.

La concentration de la silice, exprimée en sec, dans ladite suspension peut être telle que cette dernière présente un extrait sec de l'ordre de 10 à 45%.

Le traitement par le composé organique, de la silice présente au sein de ladite suspension peut être réalisé par mise en contact, sous agitation mécanique, de la suspension et du composé organique de traitement, à une température de l'ordre de 10°C à 80°C, de préférence de l'ordre de 15°C à 40°C, à un pH de l'ordre de 3 à 10, de préférence de l'ordre de 5 à 8.

La suspension de silice traitée obtenue peut, éventuellement après délitage, être ensuite séchée, afin de présenter un taux d'humidité inférieur à 10%, de préférence de l'ordre de 4 à 8%, puis broyé si nécessaire par tout moyen jusqu'à l'obtention du produit fini de granulométrie désirée.

Selon un cinquième mode de réalisation de l'invention, la silice à traiter se présente au sein d'une suspension concentrée obtenue par redispersion, dans une solution aqueuse du composé organique de traitement, de la silice sèche préalablement préparée, cette silice sèche pouvant être une silice précipitée ou un gel de silice ; le traitement par le composé organique, de la silice présente au sein de ladite suspension peut être réalisé sous agitation mécanique à une température de l'ordre de 10°C à 80°C, de préférence de l'ordre de 15°C à 40°C, à un pH de l'ordre de 3 à 10, de préférence de l'ordre de 5 à 8.

Il est tout particulièrement intéressant de mettre en oeuvre les quatrième et cinquième mode de réalisation en préliminaire de la préparation de la formulation dentifrice ; la silice traitée peut alors ne pas être séchée.

Quel que soit le mode de réalisation choisi, les opérations éventuelles de broyage peuvent être réalisées par tout moyen (à l'aide de broyeurs à marteaux, par micronisation ...), jusqu'à une granulométrie de l'ordre de 1 à 60 μm, de préférence de l'ordre de 3 à 20 μm.

Parmi les composés organiques susceptibles de traiter la silice selon l'invention pour améliorer la compatibilité de celle-ci avec les arômes, on peut citer les composés organiques non-ioniques suivants

- les polyéthylène glycols
- les alcools polyvinyliques

- les polyvinyl-pyrrolidones
- les potydiméthylsiloxanes polyalkoxylés (SILICONE COPOLYOL 10 646 de RHONE-POULENC)
- les esters de l'acide parahydroxybenzoïque

Les quantités de composé organique de traitement mises en oeuvre selon l'invention, peuvent être de l'ordre de 0,1 à 30 parties en poids, de préférence de l'ordre de 0,5 à 25 parties en poids pour 100 parties en poids de silice exprimées en matière sèche.

La silice traitée obtenue selon le procédé faisant l'objet de l'invention, présente une grande compatibilité avec les arômes, notamment avec les arômes présents dans les dentifrices.

Parmi les arômes compatibles avec les silices traitées selon l'invention, on peut mentionner tout particulièrement les arômes élémentaires couramment utilisés dans les dentifrices, tels que le menthol, la L-carvone, le salicylate de méthyle, l'aldéhyde cinamique, l'eugénol, l'anéthol, les terpènes (limonène, pinène) ..., les huiles essentielles telles que les extraits de menthe poivrée ou crépue, les extraits de badiane, girofle, cannelle, eucalyptus ...

La silice de l'invention, conservant en outre

- une grande compatibilité avec les agents thérapeutiques, le fluor notamment, ainsi qu'avec les agents anti-tartre
- des propriétés abrasives et/ou épaississantes importantes
- un indice de réfraction de l'ordre de 1,44 à 1,46,

est tout particulièrement adaptée à la formulation de pâtes dentifrices, de gels dentifrices ou de dentifrices liquides.

Ces compositions dentifrices peuvent, outre la silice de l'invention et les arômes ci-dessus mentionnés, renfermer d'autres ingrédients habituels, en particulier des agents abrasifs minéraux autres, insolubles dans l'eau, des agents épaississants, des humectants, ...

Comme agents abrasifs autres, on peut mentionner en particulier le carbonate de calcium, l'alumine hydratée, la bentonite, le silicate d'aluminium, le silicate de zirconium, les métaphosphates et phosphates de sodium, de potassium, de calcium et de magnésium. La quantité totale de poudre(s) abrasive(s) peut constituer de l'ordre de 5 à 50% du poids de la composition dentaire.

Parmi les agents épaississants, on peut mentionner tout particulièrement les silices épaississantes en quantité de l'ordre de 1 à 15 % du poids, la gomme xanthane, la gomme guar, les carraghénanes, les dérivés de la cellulose, les alginates, en quantité pouvant aller jusqu'à 5% du poids de ladite composition ....

Parmi les agents humectants, on peut citer par exemple le glycérol, le sorbitol, les polyethylène glycols, les polypropylène glycols, le xylitol, en quantité de l'ordre de 2 à 85%, de préférence de l'ordre de 10 à 70% du poids de composition dentifrice exprimé en sec.

Ces compositions dentifrices peuvent en outre comporter des agents tensio-actifs, des agents détergents, des colorants, des anti-bactériens, des dérivés fluorés, des opacifiants, des édulcorants, des agents antitartre, antiplaque, des agents de blanchiment, du bicarbonate de sodium, des antiseptiques, des enzymes ...

Les exemples suivants sont donnés à titre illustratif.

Mesure de la compatibilité vis-à-vis du fluorure de sodium NaF

Le principe de la mesure consiste à laisser en contact la silice à tester avec une solution de fluorure de sodium de concentration connue, pendant 24 heures à 37°C.

La quantité de fluorure présent dans le milieu liquide obtenu par centrifugation, est mesuré par ionométrie.

- Mode opératoire -

La silice à tester est mise en suspension à 20% dans une solution aqueuse de NaF à 1250 ppm exprimés en fluorure $F^-$ ; le rapport $F^- / SiO_2$ est donc de 5000 ppm. Le contact est maintenu 24 h à 37°C sous agitation. La suspension est centrifugée. Le surnageant est dilué 20 fois à l'eau. La concentration en F- est mesurée par ionométrie et comparée à la concentration de la solution initiale n'ayant eu aucun contact avec la silice. La quantité de $F^-$ adsorbée par la silice (exprimée en partie de $F^-$ par million de silice) est calculée.

Mesure de la compatibilité vis-à-vis des fluorures d'amine

Le principe de la mesure consiste à laisser en contact la silice à tester avec une solution de fluorure d'aminé de concentration connue, pendant 24 heures à 37°C.

La quantité de fluorure d'aminé présent dans le milieu liquide obtenu par centrifugation, est mesuré par turbidimétrie par réaction avec un réactif anionique (formation de micelles entre le fluorure d'amine et le réactif anionique mis en oeuvre pour le test).

- Mode opératoire -

*Mesure de turbidimétrie sur la solution échantillon*

On met en contact 6g de silice dans 24g d'une solution standard d'A.D.H.F. contenant 1,65% en poids de bis (hydroxyéthyl) aminopropyl N(hydroxyéthyloctadécylamine) dihydrofluorure dans le propane diol-1,2, à un pH de 5, pendant 24 heures à 37°C sous agitation magnétique.

Après centrifugation, on prélève 2g (poids £ de prise d'essai) de surnageant, qui est filtré et additionné de 5g d'eau déminéralisée.

La quantité de fluorure d'amine présente dans le milieu liquide est évaluée par turbidimétrie, en mesurant la le volume d'AEROSOL OT (solution aqueuse à 1,9g/l de dioctyl sulfosuccinate de sodium, commercialisé par TOUZARD et MATIGNON), pour faire chuter le potentiel de 1000 à 50mV (formation de micelles).

* Mesure de turbidimétrie sur la solution étalon*

On réalise de la même façon, à l'aide de l'AEROSOL OT, des mesures de turbidimétrie sur des solutions étalon contenant $\underline{M}$ grammes de solution standard d'A.D.H.F., soit respectivement 0,5g, 1g et 2g de solution standard d'A.D.H.F., dans respectivement 49,5g, 49g, et 48g d'eau déminéralisée (solutions étalon correspondant à des compatibilités de 25, 50 et 100%).

On retient comme solution étalon, celle pour laquelle le volume $\underline{Vn}$ d'AEROSOL OT coulé est le plus proche du volume $\underline{V}$ coulé pour la solution échantillon.

La compatibilité de la silice testée pour le fluorure d'amine est donnée par la formule

$$\text{Compatibilité en \%} = [(V \times M) / (Vn \times P)] \times 100$$

Mesure de la compatibilité vis-à-vis du chlorure de cétyl pyridinium (CPC)

On introduit 8g de silice à tester dans 40g d'une solution aqueuse (eau permutée) à 1,19% en poids de CPC, afin d'obtenir une suspension contenant 1% en poids de CPC. Le pH de la suspension est ajusté à 6, par ajout d'HCl ou de NaOH 2N.

La suspension est laissée pendant 24h à 37°C, sous agitation ; elle est ensuite centrifugée pendant 30minutes à 10 000 tours/minute, puis le surnageant est filtré sur un filtre de 0,22µm.

Le filtrat obtenu est dilué (1g de filtrat pour 99g d'eau) pour en mesurer l'absorbance en UV à 259 nm.

Une solution témoin de CPC est obtenue selon le même processus, sans introduction de silice.

La compatibilité de la silice testée pour le chlorure de cétyl pyridinium est donnée par la formule

$$\text{Compatibilité en \%} = \text{D.O. de l'essai} / \text{D.O. du témoin} \times 100$$

D.O. représentant la densité optique de la solution à 259 nm.

**Exemple 1**

Une bouillie réactionnelle "B", dont les caractéristiques sont données ci-après, est préparée à partir

- d'une solution aqueuse de silicate de sodium de rapport molaire $SiO_2/Na_2O$ de 3,6 ayant une concentration en $SiO_2$ de 136g/l
- et d'une solution aqueuse contenant 80g/l d'acide sulfurique.

La température en fin de précipitation est de l'ordre de 90°C.

La bouillie "B" est ensuite filtrée et lavée à l'aide d'un filtre rotatif sous vide.

Le gâteau de silice obtenu est ensuite fluidifié par simple action mécanique ; le gâteau obtenu "G" est atomisé au moyen d'un atomiseur à turbines ; le produit séché est enfin broyé ; on obtient une silice "S".

Les caractéristiques des produits correspondant aux différentes étapes sont données au tableau 1.

La prise d'huile DOP a été déterminée selon la norme ISO 787/5 en mettant en oeuvre du dioctylphtalate.

Le diamètre médian en poids $d_{50}$ des particules de silice a été déterminé à l'aide d'un appareil SYMPATEC HELOS. Cet appareil applique le principe de la diffraction FRAUNHOFFER et met en oeuvre un laser He/Ne de faible puissance. L'échantillon est préalablement dispersé par application d'ultrasons dans l'eau pendant 30 secondes pour obtenir une suspension aqueuse.

**Exemple 2**

On prélève une fraction de la bouillie "B" en fin de précipitation.
Cette fraction est ensuite divisée en deux parties "B1" et "B2", qui sont traitées comme suit:

- traitement de "B1"
  une solution aqueuse de PVP (polyvinylpyrrolidone K 30 de International Specialty Polymers) à 15 % en poids est introduite lentement sous agitation dans la bouillie "B1" maintenue à sa température d'obtention, en quantité suffisante pour obtenir un rapport PVP sur silice de 2% ;
- traitement de "B2"
  du SILICONE COPOLYOL 10646 (10646 - polyorganosiloxane polyalkoxylé de RHONE-POULENC) est introduit lentement sous agitation dans la bouillie "B2" maintenue à sa température d'obtention, en quantité suffisante pour obtenir un rapport SILICONE COPOLYOL 10646 sur silice de 2%;
  Chaque bouillie traitée est ensuite filtrée et lavée à l'eau sur une toile. Le gâteau de filtration est récupéré et séché à l'aide d'un atomiseur de laboratoire. On obtient ainsi les silices traitées "SB1" et "SB2".
  Les caractéristiques de ces silices ainsi traitées figurent au tableau 1.

**Exemple 3** ne faisant pas partie de l'invention

On prélève une fraction de gâteau de filtration "G".
Cette fraction est ensuite divisée en deux parties "G1" et "G2", qui sont traitées comme suit :

- traitement de "G1"
  on ajoute sous forte agitation du MIRAPOL A15®, solution aqueuse à 65% en poids de polymère cationique commercialisé par RHONE-POULENC, au gâteau de filtration "G1", à température ambiante, en quantité correspondant à un rapport polymère cationique / silice exprimé en matières sèches de 0,8%. L'agitation est maintenue pendant encore 5 minutes.
- traitement de "G2"
  on ajoute sous forte agitation du MIRAPOL A15®, solution aqueuse à 65% en poids de polymère cationique commercialisé par RHONE-POULENC, au gâteau de filtration "G2", à température ambiante, en quantité correspondant à un rapport polymère cationique / silice exprimé en matières sèches de 1,2%. L'agitation est maintenue pendant encore 5 minutes.
  Les gâteaux "G1" et "G2", une fois traités, sont séchés à l'aide d'un atomiseur de laboratoire . On obtient ainsi les silices traitées "SG1" et "SG2".
  Une silice témoin "SG0" est obtenue selon le même procédé, sans ajout de polymère. Les caractéristiques de ces silices ainsi traitées figurent au tableau 3.

**Exemple 4** ne faisant pas partie de l'invention

La silice "S", dont les caractéristiques sont données au tableau 1, est remise en suspension dans l'eau dans des proportions telles que l'extrait sec à 1000°C soit de l'ordre de 63 à 66%.
Cette suspension est ensuite divisée en trois parties "G3", "G4" et "G5", qui sont traitées comme suit :

- traitement de "G3"
  on ajoute sous forte agitation du GLOKILL PQ®, solution aqueuse à 50% en poids de polymère cationique (dérivé de l'épichlorhydrine et de diméthylamine) commercialisé par RHONE-POULENC, au gâteau de filtration "G3", à température ambiante, en quantité correspondant à un rapport polymère cationique / silice exprimé en matières sèches de 1%. L'agitation est maintenue pendant encore 5 minutes.
- traitement de "G4"
  on ajoute sous forte agitation du GLOKILL ELC®, solution aqueuse à 50% en poids de polymère cationique (dérivé de l'épichlorhydrine et de l'imidazole) commercialisé par RHONE-POULENC, au gâteau de filtration "G4", à température ambiante, en quantité correspondant à un rapport polymère cationique / silice exprimé en matières sèches de 1%. L'agitation est maintenue pendant encore 5 minutes.

- traitement de "G5"

on ajoute sous forte agitation une solution aqueuse à 1% de JAGUAR C162, guar cationique commercialisé par RHONE-POULENC, au gâteau de filtration "G5", à température ambiante, en quantité correspondant à un rapport guar cationique / silice exprimé en matières sèches de 1%. L'agitation est maintenue pendant encore 5 minutes.

Après traitement, les gâteaux "G3", "G4" et "G5" sont séchés en étuve ventilée pendant 18 heures à 120°C. On obtient ainsi les silices traitées"SG3", "SG4" et "SG5".

Une silice témoin "SG'0 est obtenue selon le même procédé, sans ajout de polymère. Les caractéristiques de ces silices ainsi traitées figurent au tableau 4.

**Exemple 5**

Des silices précipitées commerciales "C1", "C2", "C3", dont les caractéristiques sont données au tableau 5, sont traitées comme suit :

Dans un mélangeur en V de marque PATTERSON KELLEY, d'une contenance totale d'environ 10 litres, la solution de polymère de traitement est finement pulvérisée sur la silice, alors qu'elle est brassée mécaniquement.

La silice "C1" est divisée en trois parties "C10" (témoin), "C11" et "C12".

- traitement de "C11"

500g de cette silice sont introduits dans le mélangeur ; 500g de solution aqueuse de PVP à 5% en poids sont finement pulvérisés pendant 6 minutes, alors que la silice est brassée.

Après pulvérisation, la silice est encore maintenue en mouvement pendant 15 minutes. La silice est ensuite extraite du mélangeur et séchée en étuve pendant 24 heures à 120°C, pour éliminer l'eau excédentaire. Le rapport théorique final PVP/silice est de 5%.

- traitement de "C12"

On répète l'opération précédente à l'aide d'une solution à 5% de SILICONE COPOLYOL 10646 comme agent de traitement.

- traitement de "C10"

On répète l'opération précédente à l'aide de 500g d'eau sans agent de traitement.

La silice "C2" est divisée en trois parties "C20" (témoin). "C21" et "C22".

- traitement de "C21"

1250g de cette silice sont introduits dans le mélangeur ; 536g de solution aqueuse de PVP à 4,66% en poids sont finement pulvérisés pendant 6 minutes, alors que la silice est brassée.

Après pulvérisation, la silice est encore maintenue en mouvement pendant 15 minutes. La silice est ensuite extraite du mélangeur et séchée en étuve pendant 24 heures à 120°C, pour éliminer l'eau excédentaire. Le rapport théorique final PVP/silice est de 2%.

- traitement de "C22"

On répète l'opération précédente à l'aide d'une solution à 4,66% de SILICONE COPOLYOL 10646 comme agent de traitement.

- traitement de "C20"

On répète l'opération précédente à l'aide de 536g d'eau sans agent de traitement.

La silice "C3" est divisée en deux parties "C30" (témoin) et "C31".

- traitement de "C31"

1250g de cette silice sont introduits dans le mélangeur ; 536g de solution aqueuse de PVP à 4,66% en poids sont finement pulvérisés pendant 6 minutes, alors que la silice est brassée.

Après pulvérisation, la silice est encore maintenue en mouvement pendant 15 minutes. La silice est ensuite extraite du mélangeur et séchée en étuve pendant 24 heures à 120°C, pour éliminer l'eau excédentaire. Le rapport théorique final PVP/silice est de 2%.

- traitement de "C30"

On répète l'opération précédente à l'aide de 536g d'eau sans agent de traitement.

Les caractéristiques de ces silices ainsi traitées figurent au tableau 5.

Tableau 1

| caractéristiques | bouillie "B" | gâteau "G" | silice "S" |
|---|---|---|---|
| pH | 3,4 | 6,3 | 6,5 |
| sulfates (%) | 7,8 | 0,8 | 0,5 |

Tableau 1   (suite)

| caractéristiques | bouillie "B" | gâteau "G" | silice "S" |
|---|---|---|---|
| humidité | - | - | 6,5 |
| perte au feu | 93 | 62,4 | 10,3 |
| DOP | - | - | 100 |
| RP/DA | - | - | 100 |
| d50 | 13,4 | 25 | 27,7 |
| compatibilité CPC | - | - | 36 |
| compatibilité NaF | - | - | 88 |
| compatibilité fluorure d'amine | - | - | 45 |
| compatibilité arôme | - | - | 15-20 |

Tableau 2

| caractéristiques | SB1 | SB2 |
|---|---|---|
| pH | 7,61 | 7,5 |
| sulfates (%) | 0,06 | - |
| humidité | 4,5 | 5,8 |
| perte au feu | 9,5 | 10,7 |
| d50 | 12,1 | 13,6 |
| compatibilité CPC | 82 | 72 |
| compatibilité NaF | 92 | 94 |
| compatibilité FA | 86 | 84 |
| compatibilité arôme | 87 | 66 |

Tableau 3

| caractéristiques | SG0 | SG1 | SG2 |
|---|---|---|---|
| pH | 7,0 | 6,3 | 6,2 |
| sulfates (%) | 0,5 | 0,5 | 0,5 |
| humidité | 4,8 | 5,3 | 4,3 |
| perte au feu | 8,81 | 10,1 | 9,3 |
| d50 | 21 | 27,2 | 31,7 |
| compatibilité CPC | 42 | 60 | 65 |
| compatibilité NaF | ≈90 | 85 | 83 |
| compatibilité FA | 48 | 99 | 98 |
| compatibilité arôme | 25 | 47 | 45 |

Tableau 4

| caractéristiques | SG'0 | SG3 | SG4 | SG5 |
|---|---|---|---|---|
| pH | 7,0 | 6,2 | 6,3 | 6,5 |

Tableau 4   (suite)

| caractéristiques | SG'0 | SG3 | SG4 | SG5 |
|---|---|---|---|---|
| sulfates (%) | 1,0 | 0,8 | 0,7 | 0,8 |
| humidité | 0 | 0 | 0 | 0 |
| perte au feu | 3,12 | 4,3 | 4,4 | 4,3 |
| d50 | 31,2 | 34,6 | 31,8 | 32,7 |
| compatibilité CPC | 26 | 36 | 42 | 43 |
| compatibilité NaF | 91 | 89 | 84 | 89 |
| compatibilité FA | 34 | 58 | 87 | 51 |
| compatibilité arôme | 28 | 62 | 54 | 63 |

Tableau 5

| caractéristiques | C1 | C10 | C11 | C12 | C2 | C20 | C22 | C22 | C3 | C30 | C31 |
|---|---|---|---|---|---|---|---|---|---|---|---|
| pH | 7,3 | 7,6 | 7,9 | 7,5 | 6,6 | 7,0 | 7,4 | 4,3 | 6,9 | 6,8 | 7,2 |
| sulfates (%) | 2,7 | 2,8 | 2,4 | 2,6 | 0,6 | 0,6 | 0,6 | 0,7 | 0,3 | 0,3 | 0,3 |
| humidité | 7,1 | 3,4 | 15,9 | 7,8 | 5,9 | 1,5 | 8,1 | 10,4 | 3,6 | 0,6 | 7,9 |
| perte au feu | 11,0 | 6,9 | 22,4 | 15,0 | 9,7 | 5,2 | 13,3 | 15,4 | 6,2 | 3,3 | 12,1 |
| DOP | 344 | 236 | 268 | 304 | 90 | 90 | 92 | 88 | 124 | 148 | 116 |
| RP/DA | <10 | <10 | <10 | <10 | 100 | 92 | 66 | 80 | 80 | 80 | 75 |
| d50 | 11 | 12 | 11 | 11 | 8 | 8 | 8 | 7 | 10 | 10 | 10 |
| compatibilité CPC | - | - | - | - | 28 | 23 | 64 | 58 | - | 16 | 47 |
| compatibilité FA | - | - | - | - | 53 | 52 | 96 | 68 | 15 | 10 | 67 |
| compatibilité NaF | 92 | 94 | 94 | 96 | 94 | 96 | 96 | 96 | 95 | 98 | 97 |
| compatibilité arôme | 0 | 2 | 16 | 23 | 6 | 3 | 41 | 50 | 1 | 3 | 30 |

**Revendications**

1. Procédé de préparation de la silice de compatibilité améliorée avec les arômes présentant pour une abrasivité RP/DA inférieure ou égale à 10, une compatibilité avec les arômes supérieure à 10%, de préférence supérieure à 15%, et pour une abrasivité RP/DA supérieure à 10, une compatibilité avec les arômes d'au moins 30%, de préférence supérieure à 50%, par traitement, à l'aide d'un composé organique, de la surface d'une silice issue de la réaction d'un silicate de métal alcalin avec un agent acide minéral ou organique, silice se présentant sous forme sèche ou au sein d'une suspension aqueuse
ledit procédé étant **caractérisé en ce que** ledit composé organique est un composé organique hydrosoluble non-ionique et **en ce que** ledit traitement est réalisé par développement de liaisons hydrogène entre les groupements silanols SiOH de la surface de la silice à traiter et ledit composé organique non-ionique, ladite silice traitée étant ensuite si nécessaire séparée et/ou lavée et/ou séchée.

2. Procédé selon la revendication 1), **caractérisé en ce que** la silice à traiter se présente au sein de la suspension diluée constituée par la bouillie issue directement de la réaction du silicate de métal alcalin avec l'agent acide minéral ou organique.

3. Procédé selon la revendication 2), **caractérisé en ce que** le traitement par le composé organique est réalisé par mise en contact, sous agitation mécanique, de la bouillie réactionnelle et du composé organique de traitement, à une température de l'ordre de 20°C à 100°C, à un pH de l'ordre de 1 à 10, de préférence de l'ordre de 3 à 6.

**4.** Procédé selon la revendication 4), **caractérisé en ce que** la silice à traiter se présente au sein de la suspension concentrée constituée par le gâteau de silice issu de la filtration, avec éventuellement lavage à l'eau, de la bouillie réactionnelle formée par réaction du silicate de métal alcalin avec l'agent acide minéral ou organique.

**5.** Procédé selon la revendication 4), **caractérisé en ce que** le traitement, par le composé organique, de la silice présente au sein du gâteau de filtration est réalisé par mise en contact, sous agitation mécanique, du gâteau de filtration et du composé organique de traitement, à une température de l'ordre de 10°C à 80°C, de préférence de l'ordre de 15°C à 40°C, à un pH de l'ordre de 3 à 10, de préférence de l'ordre de 5 à 8.

**6.** Procédé selon la revendication 1), **caractérisé en ce que** la silice à traiter par le composé organique est une silice sous forme sèche présentant une humidité inférieure à 50%, de préférence inférieure à 10%, obtenue par séchage, éventuellement après délitage, du gâteau de silice issu de la filtration avec lavage de la bouillie réactionnelle formée par réaction du silicate de métal alcalin avec l'agent acide minéral ou organique.

**7.** Procédé selon la revendication 6), **caractérisé en ce que** le traitement par le composé organique est effectué par pulvérisation du composé organique de traitement sur la surface de la silice sèche.

**8.** Procédé selon la revendication 1), **caractérisé en ce que** la silice à traiter se présente au sein d'une suspension concentrée obtenue par redispersion dans l'eau d'une silice sèche préalablement préparée.

**9.** Procédé selon la revendication 8), **caractérisé en ce que** le traitement par le composé organique, de la silice présente au sein de ladite suspension est réalisé par mise en contact, sous agitation mécanique, de la suspension et du composé organique de traitement, à une température de l'ordre de 10°C à 80°C, de préférence de l'ordre de 15°C à 40°C, à un pH de l'ordre de 3 à 10, de préférence de l'ordre de 5 à 8.

**10.** Procédé selon la revendication 1), **caractérisé en ce que** la silice à traiter se présente au sein d'une suspension concentrée obtenue par redispersion, dans une solution aqueuse du composé organique de traitement, de la silice sèche préalablement préparée,

**11.** Procédé selon la revendication 10), **caractérisé en ce que** le traitement par le composé organique, de la silice présente au sein de ladite suspension est réalisé sous agitation mécanique à une température de l'ordre de 10°C à 80°C, de préférence de l'ordre de 15°C à 40°C, à un pH de l'ordre de 3 à 10, de préférence de l'ordre de 5 à 8.

**12.** Procédé selon l'une quelconque des revendications 1) à 11), **caractérisé en ce que** les composés organiques sont

- les polyéthylène glycols
- les alcools polyvinyliques
- les polyvinyl-pyrrolidones
- les polydiméthylsiloxanes polyalkoxylés
- les esters de l'acide parahydroxybenzoïque

**13.** Procédé selon l'une quelconque des revendications 1) à 12), **caractérisé en ce que** les quantités de composé organique de traitement mises en oeuvre sont de l'ordre de 0,1 à 30 parties en poids, de préférence de l'ordre de 0,5 à 25 parties en poids pour 100 parties en poids de silice exprimées en matière sèche.

**14.** Utilisation de la silice obtenue selon le procédé faisant l'objet de l'une quelconque des revendications 1) à 13), comme agent abrasif ou épaississant dans les compositions dentifrices.

**15.** Composition dentifrice comprenant la silice obtenue selon le procédé faisant l'objet de l'une quelconque des revendications 1) à 13).

**Patentansprüche**

**1.** Verfahren zur Herstellung einer Kieselsäure mit verbesserter Kompatibilität in bezug auf Aromastoffe, welche bei einer RP/DA-Abrasivität (Abriebvermögen) von weniger oder gleich 10 eine Kompatibilität in bezug auf Aromastoffe von mehr als 10 %, vorzugsweise von mehr als 15 %, und bei einer RP/DA-Abrasivität von mehr als 10 eine Kompatibilität in bezug auf Aromastoffe von mindestens 30 %, vorzugsweise von mehr als 50 %, aufweist, durch Behandlung der Oberfläche einer aus der Reaktion eines Alkalimetallsilikats mit einem sauren organischen oder anorganischen Reagenz stammenden Kieselsäure mittels einer organischen Verbindung, wobei die Kieselsäure in trockener Form oder in einer wäßrigen Suspension vorliegt,
**dadurch gekennzeichnet,**
**daß** die organische Verbindung eine nichtionische wasserlösliche organische Verbindung ist und daß die Behandlung durch Ausbildung von Wasserstoffbindungen zwischen den Silanolgruppen SiOH der Oberfläche der zu behandelnden Kieselsäure und der nichtionischen organischen Verbindung erfolgt, wobei die behandelte Kieselsäure anschließend, falls erforderlich, abgetrennt und/oder gewaschen und/oder getrocknet wird.

2. Verfahren nach Anspruch 1, **dadurch gekennzeichnet, daß** die zu behandelnde Kieselsäure in einer verdünnten Suspension vorliegt, die aus der Brühe besteht, die direkt aus der Reaktion des Alkalimetallsilikats mit dem sauren organischen oder anorganischen Reagenz stammt.

3. Verfahren nach Anspruch 2, **dadurch gekennzeichnet, daß** die Behandlung mit der organischen Verbindung durch Inkontaktbringen der Reaktionsbrühe mit der der Behandlung dienenden organischen Verbindung unter mechanischem Rühren bei einer Temperatur von etwa 20 °C bis etwa 100 °C bei einem pH-Wert von etwa 1 bis etwa 10, vorzugsweise etwa 3 bis etwa 6, erfolgt.

4. Verfahren nach Anspruch 1, **dadurch gekennzeichnet, daß** die zu behandelnde Kieselsäure in einer konzentrierten Suspension vorliegt, die aus dem Kieselsäurekuchen besteht, der aus der Filtration mit gegebenenfalls durchgeführtem Waschen der Reaktionsbrühe, die durch Reaktion des Alkalimetallsilikats mit dem sauren organischen oder anorganischen Reagenz gebildet wird, mit Wasser stammt.

5. Verfahren nach Anspruch 4, **dadurch gekennzeichnet, daß** die Behandlung der in dem Filterkuchen vorliegenden Kieselsäure mit der organischen Verbindung durch Inkontaktbringen des Filterkuchens mit der der Behandlung dienenden organischen Verbindung unter mechanischem Rühren bei einer Temperatur von etwa 10 °C bis etwa 80 °C, vorzugsweise etwa 15 °C bis etwa 40 °C, bei einem pH-Wert von etwa 3 bis etwa 10, vorzugsweise etwa 5 bis etwa 8, erfolgt.

6. Verfahren nach Anspruch 1, **dadurch gekennzeichnet, daß** die mit der organischen Verbindung zu behandelnde Kieselsäure eine Kieselsäure in trokkener Form mit einer Feuchtigkeit von weniger als 50 %, vorzugsweise weniger als 10 %, ist, die durch Trocknung, gegebenenfalls nach Zerkleinern, des Kieselsäurekuchens erhalten wird, der aus der Filtration mit Waschen der Reaktionsbrühe, die durch Reaktion des Alkalimetallsilikats mit dem sauren organischen oder anorganischen Reagenz gebildet wird, stammt.

7. Verfahren nach Anspruch 6, **dadurch gekennzeichnet, daß** die Behandlung mit der organischen Verbindung durch Pulverisierung (Zerstäubung) der der Behandlung dienenden organischen Verbindung auf der Oberfläche der trockenen Kieselsäure erfolgt.

8. Verfahren nach Anspruch 1, **dadurch gekennzeichnet, daß** die zu behandelnde Kieselsäure in einer konzentrierten Suspension vorliegt, die durch erneute Dispergierung einer zuvor hergestellten Kieselsäure in Wasser erhalten wird.

9. Verfahren nach Anspruch 8, **dadurch gekennzeichnet, daß** die Behandlung der in der Suspension vorliegenden Kieselsäure mit der organischen Verbindung durch Inkontaktbringen der Suspension mit der der Behandlung dienenden organischen Verbindung unter mechanischem Rühren bei einer Temperatur von etwa 10 °C bis etwa 80 °C, vorzugsweise etwa 15 °C bis etwa 40 °C, bei einem pH-Wert von etwa 3 bis etwa 10, vorzugsweise etwa 5 bis etwa 8, durchgeführt wird.

10. Verfahren nach Anspruch 1, **dadurch gekennzeichnet, daß** die zu behandelnde Kieselsäure in einer konzentrierten Suspension vorliegt, die durch erneute Dispergierung der zuvor hergestellten trockenen Kieselsäure in einer wäßrigen Lösung der der Behandlung dienenden organischen Verbindung durchgeführt wird.

11. Verfahren nach Anspruch 10, **dadurch gekennzeichnet, daß** die Behandlung der in der Suspension befindlichen Kieselsäure mit der organische Verbindung unter mechanischem Rühren bei einer Temperatur von etwa 10 °C bis etwa 80 °C, vorzugsweise etwa 15 °C bis etwa 40 °C, bei einem pH-Wert von etwa 3 bis etwa 10, vorzugsweise etwa 4 bis etwa 8, durchgeführt wird.

**12.** Verfahren nach einem der Ansprüche 1 bis 11, **dadurch gekennzeichnet, daß** die organischen Verbindungen

- Polyethylenglykole,

- Polyvinylalkohole,

- Polyvinylpyrrolidone,

- polyalkoxylierte Polydimethoxysilane,

- Parahydroxybenzoesäureester

sind.

**13.** Verfahren nach einem der Ansprüche 1 bis 12, **dadurch gekennzeichnet, daß** die Mengen an zur Behandlung eingesetzter organischer Verbindung etwa 0,1 bis etwa 30 Gewichtsteile, vorzugsweise etwa 0,5 bis etwa 25 Gewichtsteile, auf 100 Gewichtsteile Kieselsäure betragen, berechnet als Trockenmaterial.

**14.** Verwendung der gemäß dem Verfahren nach einem der Ansprüche 1 bis 13 erhaltenen Kieselsäure als Abrasionsmittel (Schleif-/Poliermittel) oder als Verdickungsmittel in Zahnpflegemittelzusammensetzungen.

**15.** Zahnpflegemittelzusammensetzung, enthaltend die gemäß dem Verfahren nach einem der Ansprüche 1 bis 13 erhaltene Kieselsäure.


**Claims**

**1.** Process for preparing silica of improved compatibility with flavourings, having, for an RP/DA abrasiveness of less than or equal to 10, a compatibility with flavourings of greater than 10% and preferably greater than 15%, and for an RP/DA abrasiveness of greater than 10, a compatibility with flavourings of at least 30% and preferably greater than 50%, by treatment, using an organic compound, of the surface of a silica obtained from the reaction of an alkali metal silicate with a mineral or organic acid agent, the said process being **characterized in that** the said organic compound is nonionic and **in that** the said treatment is performed by developing hydrogen bonds between the silanol groups SiOH of the surface of the silica to be treated and the said nonionic organic compound, the said treated silica then being, if necessary, separated out and/or washed and/or dried.

**2.** Process according to Claim 1, **characterized in that** the silica to be treated is in the dilute suspension consisting of the slurry obtained directly from the reaction of the alkali metal silicate with the mineral or organic acid agent.

**3.** Process according to Claim 2, **characterized in that** the treatment with the organic compound is performed by placing the reaction slurry and the organic treatment compound in contact, with mechanical stirring, at a temperature from about 20°C to 100°C, and at a pH from about 1 to 10 and preferably from about 3 to 6.

**4.** Process according to Claim 3, **characterized in that** the silica to be treated is in the concentrated suspension consisting of the silica cake obtained from the filtration, optionally while washing with water, of the reaction slurry formed by the reaction of the alkali metal silicate with the mineral or organic acid agent.

**5.** Process according to Claim 4, **characterized in that** the treatment, with the organic compound, of the silica in the filter cake is performed by placing the filter cake and the organic treatment compound in contact, with mechanical stirring, at a temperature from about 10°C to 80°C and preferably from about 15°C to 40°C, and at a pH from about 3 to 10 and preferably from about 5 to 8.

**6.** Process according to Claim 1, **characterized in that** the silica to be treated with the organic compound is a silica in dry form having a moisture content of less than 50% and preferably less than 10%, obtained by drying, optionally after crumbling, of the silica cake obtained from the filtration with washing of the reaction slurry formed by reaction of the alkali metal silicate with the mineral or organic acid agent.

**7.** Process according to Claim 6, **characterized in that** the treatment with the organic compound is carried out by spraying the organic treatment compound onto the surface of the dry silica.

8. Process according to Claim 1, **characterized in that** the silica to be treated is in a concentrated suspension obtained by redispersion in water of a dry silica prepared beforehand.

9. Process according to Claim 8, **characterized in that** the treatment with the organic compound of the silica in the said suspension is performed by placing the suspension and the organic treatment compound in contact, with mechanical stirring, at a temperature from about 10°C to 80°C and preferably from about 15°C to 40°C, and at a pH from about 3 to 10 and preferably from about 5 to 8.

10. Process according to Claim 1, **characterized in that** the silica to be treated is in a concentrated suspension obtained by redispersion, in an aqueous solution of the organic treatment compound, of the dry silica prepared beforehand.

11. Process according to Claim 10, **characterized in that** the treatment with the organic compound of the silica in the said suspension is performed with mechanical stirring at a temperature from about 10°C to 80°C and preferably from about 15°C to 40°C, and at a pH from about 3 to 10 and preferably from about 5 to 8.

12. Process according to any one of Claims 1 to 11, **characterized in that** the organic compounds are

   - polyethylene glycols
   - polyvinyl alcohols
   - polyvinylpyrrolidones
   - polyalkoxylated polydimethylsiloxanes
   - para-hydroxybenzoic acid esters.

13. Process according to any one of Claims 1 to 12, **characterized in that** the amounts of organic treatment compound used are from about 0.1 to 30 parts by weight and preferably from about 0.5 to 25 parts by weight per 100 parts by weight of silica, expressed as solids.

14. Use of the silica obtained according to the process forming the subject of any one of Claims 1 to 13, as an abrasive agent or thickener in toothpaste compositions.

15. Toothpaste composition comprising silica obtained according to the process forming the subject of any one of Claims 1 to 13.